Europäisches Patentamt

European Patent Office

Office européen des brevets

(19)

(11) Veröffentlichungsnummer: **0 005 275**
**B1**

(12) # EUROPÄISCHE PATENTSCHRIFT

(45) Veröffentlichungstag der Patentschrift:
22.12.82

(51) Int. Cl.³: **A 61 F 1/00**

(21) Anmeldenummer: 79101377.4

(22) Anmeldetag: 05.05.79

(54) **Brustprothese.**

(30) Priorität: 08.05.78 DE 2819968

(43) Veröffentlichungstag der Anmeldung:
14.11.79 Patentblatt 79/23

(45) Bekanntmachung des Hinweises auf die Patenterteilung:
22.12.82 Patentblatt 82/51

(84) Benannte Vertragsstaaten:
AT BE CH DE FR GB IT NL SE

(56) Entgegenhaltungen:
DE-A-2 604 744
DE-A-2 639 032
DE-U-1 739 612
DE-U-7 440 175
DE-U-7 813 097
DE-U-7 814 123
US-A-2 543 499
US-A-3 911 503

(73) Patentinhaber: Bauerfeind, Hans B., Wiesenstrasse 18,
D-4152 Kempen - 1 (DE)

(72) Erfinder: Bauerfeind, Hans B., Wiesenstrasse 18,
D-4152 Kempen - 1 (DE)

(74) Vertreter: Stark, Walter, Dr.-Ing., Moerser Strasse 140,
D-4150 Krefeld (DE)

Brustprothese

Die Erfindung betrifft eine Brustprothese mit einem weichen, der natürlichen Brust nachgebildeten Prothesenkörper aus einem Kunststoff mit Rückstellelastizität, insbesondere Silikonkautschuk, wobei an der Rückseite des Prothesenkörpers eine festere Kunststoffschicht angeordnet ist, die eine Aushöhlung begrenzt.

Wenn eine Frau Brustkrebs hat, ist die operative Entfernung der Brust oftmals die einzige wirksame Gegenmassnahme. Das Fehlen der Brust bewirkt bei den Operierten dann oft eine Minderung des Selbstwertgefühles und andere seelische Behinderungen. Um dies zu verhindern, wurden Brustprothesen entwickelt, wobei man bemüht ist, die Eigenschaften einer natürlichen Brust möglichst weitgehend zu verwirklichen.

Als ein geeignetes Material für derartige Brustprothesen hat sich Silikonkautschuk erwiesen, da er auf eine der natürlichen Brust weitgehend entsprechende Rückstellelastizität, auch Formgedächtnis genannt, einstellbar ist und auch hinsichtlich der Weichheit dem natürlichen Vorbild angepasst werden kann.

Der Nachteil dieses Materials besteht darin, dass es, verglichen mit einer natürlichen Brust, als zu schwer empfunden wird. Aus diesem Grunde hat man auf der dem Körper zugewandten Rückseite der Brustprothese eine Aushöhlung vorgesehen. Durch diese Aushöhlung konnte zwar das Gewicht entsprechend angepasst werden, ihre Form behält sie jedoch nur dann bei, wenn ein wesentlich härter eingestellter Silikonkautschuk verwendet wird, so dass die der natürlichen Brust entsprechende Weichheit verlorengeht.

Ferner ist eine Brustprothese bekannt (DE-A-26 04 744), bei der der Prothesenkörper zwischen zwei Kunststoff-Folien eingebettet und eingeschweisst ist. Diese Kunststoff-Folien sollen hauptsächlich den weichen Silikonkautschuk schützen. Da sie aus einem festeren Material als Silikonkautschuk bestehen, verändern sie auch die Weichheit der Prothese insgesamt. Ausserdem gibt es Brustprothesen mit einer Flüssigkeitsfüllung (US-A-25 43 499).

In vielen Fällen ist es erforderlich, eine Brustprothese nachträglich den gegebenen Formen anzupassen. Das ist mit den bekannten Ausführungsformen nicht möglich.

Der Erfindung liegt deswegen die Aufgabe zugrunde, eine Brustprothese der eingangs beschriebenen Gattung so zu verbessern, dass sie hinsichtlich ihrer Eigenschaften, insbesondere der Weichheit, einer natürlichen Brust möglichst nahekommt, wobei die Möglichkeit gegeben sein soll, die Brustprothese im Einzelfall anzupassen.

Diese Aufgabe wird dadurch gelöst, dass die festere Kunststoffschicht lediglich an der Rückseite des Prothesenkörpers und als gegossenes Formstück ausgebildet ist, das eine für die Formstabilisierung der Aushöhlung ausreichende Festigkeit aufweist und aussenseitig den Prothesenkörper trägt, und dass das gegossene Formstück am angrenzenden Rand des Prothesenkörpers übersteht.

Diese Brustprothese erfüllt alle Eigenschaften, insbesondere hinsichtlich der Weichheit, einer natürlichen Brust. Durch Beschneiden und Nachschleifen des überstehenden Randes kann die Brustprothese auch nachträglich genau an die Person, für die sie bestimmt ist, abgepasst werden.

Der Prothesenkörper kann eine Brustwarzennachbildung mit einer gegenüber dem benachbarten Kunststoff höheren Festigkeit aufweisen. Dabei ist es zweckmässig, wenn die Brustwarzennachbildung aus dem gleichen Kunststoff wie die rückseitige Schicht des Prothesenkörpers besteht. Hierdurch ist die Brustprothese auch insoweit einer natürlichen Brust weitgehend angepasst.

Ferner kann der Prothesenkörper mit einem Textilbezug versehen sein. Dieser Textilbezug schützt den Prothesenkörper, ohne dessen Eigenschaften nennenswert zu beeinträchtigen. Dabei kann der Textilbezug auf den übrigen Teil des Prothesenkörpers, der aus einem der Weichheit der natürlichen Brust angepassten Kunststoff besteht, beschränkt sein. Zwar wird dann die rückseitige Schicht nicht mit abgedeckt, da diese jedoch aus einem wesentlich härteren Stoff besteht, ist ihr Schutz nicht so erforderlich wie bei dem übrigen Teil des Prothesenkörpers. Andererseits bleibt die Möglichkeit einer Bearbeitung des Randes der rückseitigen Schicht zur Anpassung erhalten.

In der Zeichnung ist die Erfindung anhand eines Ausführungsbeispieles näher veranschaulicht. Es zeigen:

Fig. 1 eine Vorderansicht einer Brustprothese und

Fig. 2 einen Vertikalschnitt durch die Brustprothese gemäss Fig. 1.

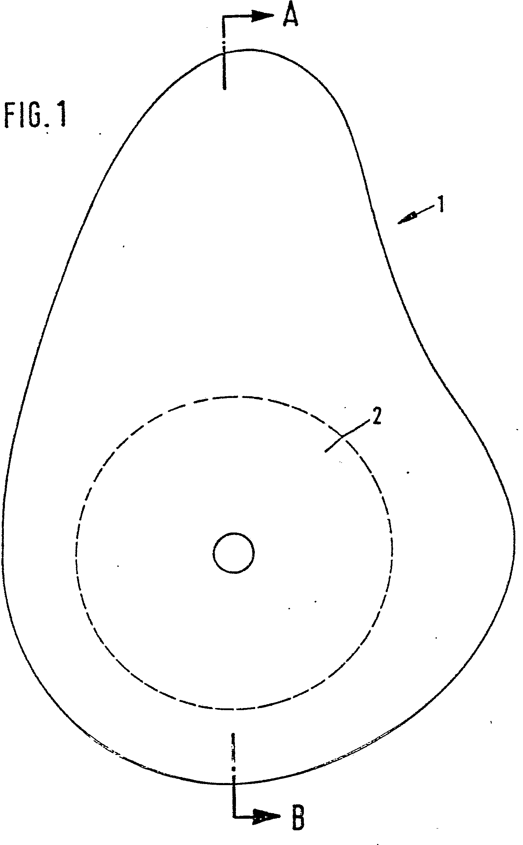

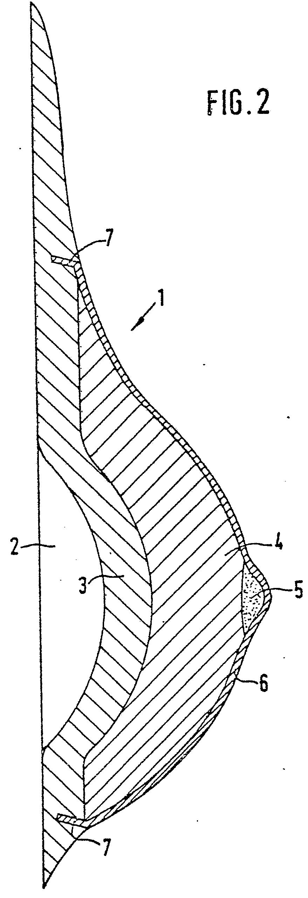

Fig. 1 und 2 zeigen eine Brustprothese, deren äussere Form einer natürlichen Brust nachgebildet ist. Lediglich auf der Rückseite ist eine Aushöhlung 2 eingeformt, die der Gewichtsreduzierung in Anpassung an eine natürliche Brust dient.

Damit die Form der Aushöhlung 2 auch hinreichend stabil ist, wird die Rückseite der Brustprothese 1 von einer Schicht 3 gebildet, die eine für diesen Zweck ausreichende Festigkeit aufweist. Auf diese Schicht 3 ist der übrige Teil 4 der Brustprothese 1 aufgeformt, der aus einem der Weichheit der natürlichen Brust entsprechenden Silikonkautschuk besteht. Zusätzlich weist dieser Teil noch eine Brustwarzennachbildung 5 auf, die aus einem Silikonkautschuk derselben Festigkeit wie der der rückseitigen Schicht 3 besteht. Die Vorderseite der Brustprothese ist zusätzlich noch mit einem schützenden Textilbezug 6 versehen. Dieser ist auf den Teil 4 sowie auf die Brustwarzennachbildung 5 der Brustprothese 1 beschränkt, da nur diese Teile eines Schutzes bedürfen. Ein über diese Teile überstehender Rand 7 des Textilbezuges 6 ist in die rückseitige Schicht 3 eingeformt, so dass der Textilbezug 6 einen sicheren Halt hat. Der Rand der rückseitigen Schicht 3 bleibt somit frei für ein nachträgliches Beschneiden und Nachschleifen zur Anpassung an die Trägerin dieser Brustprothese 1.

Für die Herstellung wird zunächst die Brustwarzennachbildung 5 und dann der weiche Teil 4 gegossen. Der überstehende Teil des Textilbezuges 6 wird dann umgebogen, so dass er beim anschliessenden Giessen der rückseitigen Schicht 3 in diese eingeformt wird.

### Patentansprüche

1. Brustprothese mit einem weichen, der natürlichen Brust nachgebildeten Prothesenkörper (4) aus einem Kunststoff mit Rückstellelastizität, insbesondere Silikonkautschuk, wobei an der Rückseite des Prothesenkörpers (4) eine festere Kunststoffschicht (3) angeordnet ist, die eine Aushöhlung (2) begrenzt, dadurch gekennzeichnet, dass die festere Kunststoffschicht (3) lediglich an der Rückkseite des Prothesenkörpers (4) und als gegossenes Formstück (3) ausgebildet ist, das eine für die Formstabilisierung der Aushöhlung (2) ausreichende Festigkeit aufweist und aussenseitig den Prothesenkörper (4) trägt, und dass das gegossene Formstück (3) am angrenzenden Rand des Prothesenkörpers (4) übersteht.·

2. Brustprothese nach Anspruch 1, dadurch gekennzeichnet, dass der Prothesenkörper eine Brustwarzennachbildung (5) mit einer gegenüber dem benachbarten Kunststoff höheren Festigkeit aufweist.

3. Brustprothese nach Anspruch 2, dadurch gekennzeichnet, dass die Brustwarzennachbildung (5) aus dem gleichen Kunststoff wie die rückseitige Schicht (3) des Prothesenkörpers besteht.

4. Brustprothese nach einem der Ansprüche 1–3, dadurch gekennzeichnet, dass der Prothesenkörper mit einem Textilbezug (6) versehen ist.

### Claims

1. A breast prosthesis with a soft prosthetic member (4), imitating the natural breast, made of a synthetic material with restoring resilience, in particular silicone rubber, on the rear of the prosthetic member (4) a relatively rigid synthetic material layer (3) being arranged which defines a hollow (2), characterized in that the relatively rigid synthetic material layer (3) is merely on the rear of the prosthetic member (4) and is constructed as a cast shape (3) which has a rigidity sufficient to stabilize the shape of the hollow (2) and externally bears the prosthetic member (4); and in that the cast shape (3) projects beyond the adjacent edge of the prosthetic member (4).

2. A breast prosthesis according to claim 1, characterized in that the prosthetic member comprises an imitation of the breast nipple (5) with a rigidity which is greater in comparison to the adjacent synthetic material.

3. A breast prosthesis according to claim 2, characterized in that the imitation of the breast nipple (5) is made of the same synthetic material as the rear layer (3) of the prosthetic member. ·

4. A breast prosthesis according to one of claims 1–3, characterized in that the prosthetic member is provided with a textile covering (6).

### Revendications

1. Prothèse du sein comprenant un corps de prothèse mou (4) reproduisant la forme d'un sein naturel, constitué en une matière synthétique à élasticité de retour en forme, en particulier du caoutchouc au silicone, une couche de matière synthétique plus solide (3) étant disposée sur la face arrière du corps de prothèse (4) pour délimiter une partie creuse 2, prothèse caractérisée en ce que la couche de matière synthétique plus rigide (3) est constituée seulement sur la face arrière du corps de prothèse (4) sous la forme d'une pièce coulée (3), qui présente une rigidité suffisante pour la stabilisation de la partie creuse (2) et supporte extérieurement le corps de prothèse (4), cette pièce de forme coulée (3) dépassant au delà du bord limite du corps de prothèse (4).

2. Prothèse de sein suivant la revendication 1, caractérisé en ce que le corps de prothèse présente une pièce simulant un mamelon de sein (5) présentant une rigidité supérieure à celle de la matière synthétique voisine (4).

3. Prothèse de sein suivant la revendication 2, caractérisé en ce que la pièce simulant un mamelon de sein (5) est constituée en la même matière synthétique que la couche de face arrière (3) du corps de prothèse.

4. Prothèse de sein suivant l'une quelconque des revendications 1 à 3, caractérisé en ce que le corps de prothèse est pourvu d'un revêtement protecteur en tissu textile (6).

FIG.1

0005275

FIG.2

7